(19) Europäisches Patentamt
European Patent Office
Office européen des brevets

(11) Numéro de publication: **0 224 897 B1**

(12) # FASCICULE DE BREVET EUROPEEN

(45) Date de publication de fascicule du brevet: **09.10.91**

(51) Int. Cl.5: **C07C 403/16**

(21) Numéro de dépôt: **86116630.4**

(22) Date de dépôt: **29.11.86**

(54) **Procédé pour la préparation de cétones cycloaliphatiques.**

(30) Priorité: **04.12.85 CH 5161/85**

(43) Date de publication de la demande:
**10.06.87 Bulletin 87/24**

(45) Mention de la délivrance du brevet:
**09.10.91 Bulletin 91/41**

(84) Etats contractants désignés:
**CH DE FR GB LI NL**

(56) Documents cités:
**CH-A- 503 684**
**CH-A- 537 352**
**US-A- 3 928 456**

**TETRAHEDRON, vol. 42, no. 12, 1986, pages 3245-3250, Pergamon Journals Ltd, Oxford, GB; F. NAEF et al.: "Grignard and hydride addition to a ketene intermediate: A novel access to alpha-damascone and alpha-cyclocitral"**

(73) Titulaire: **FIRMENICH SA**
**1, route des Jeunes**
**CH-1211 Genève 8(CH)**

(72) Inventeur: **Naef, Ferdinand, Dr.**
**30, Chemin Vert**
**CH-1227 Carouge(CH)**
Inventeur: **Decorzant, René**
**3, avenue du Gros Chêne**
**CH-1213 Onex(CH)**

(74) Mandataire: **Salvadori, Giuseppe, Dr.**
**c/o Firmenich S.A. Case Postale 239**
**CH-1211 Genève 8(CH)**

**EP 0 224 897 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

Depuis sa découverte [voir E. Demole et al., Helv. Chim. Acta 53, 541 (1970)], l'alpha-damascone s'est affirmée comme un ingrédient parfumant et aromatisant de choix. Produit d'origine naturelle, il développe une odeur rosée puissante avec une tonalité verte et fruitée qui rappelle la note odorante de la pomme verte.

A ce jour, nombreuses ont été les voies de synthèse proposées pour sa préparation. Parmi celles-ci figure celle qui fait appel à une réaction d'addition de type de Grignard entre l'alpha-cyclogéraniate de méthyle et le chlorure d'allyl-magnésium. Ce procédé présente toutefois le désavantage de donner lieu à la formation d'un carbinol diallylique suivant le schéma réactionnel A.

Pour parer à cet inconvénient, K.-H. Schulte-Elte et al. [voir brevet européen EP-B1 0070995] ont développé une méthode générale de clivage d'un alcool allylique par traitement de celui-ci avec une base forte, ce qui leur a permis de transformer le carbinol diallylique III en la cétone IIa, précurseur direct de l'alpha-damascone. C. Fehr [voir brevet européen EP-B1 0093840] a montré que l'addition d'un halogénure d'allyl-magnésium sur l'alpha-cyclogéraniate de méthyle en présence d'une base forte, tel l'amidure de diisopropyl lithium, conduisait à la formation de la cétone IIa qui était rapidement déprotonée en l'énolate de formule (V).

L'attaque ultérieure d'une deuxième molécule d'halogénure d'allyl-magnésium était ainsi évitée. L'hydrolyse de l'énolate, suivie d'une isomérisation acide donnait lieu à la formation de l'alpha-damascone désirée.

La présente invention apporte une solution nouvelle et originale au problème posé par la préparation de l'alpha-damascone. L'invention est définie comme indiqué aux revendications; elle a plus particulièrement pour objet un procédé qui consiste en l'addition d'un halogénure d'alkényl-magnésium de formule (VI) contenant une double liaison dans l'une des positions indiquées par les pointillés et dans laquelle X représente un atome d'halogène sur un cétène de formule (I) et en l'hydrolyse du produit résultant pour fournir la cétone cycloaliphatique de formule (IIa,b) contenant une double liaison dans l'une des positions indiquées par les pointillés.

Le symbole X dans la formule de l'halogènure d'alkényl-magnésium peut représenter un atome de chlore, brome ou iode.

Le procédé de l'invention a recours à des réactifs et des produits de départ aisément disponibles et bon marché et se révèle de ce fait avantageux. Ce procédé est également caractérisé par des étapes au mode opératoire simple et peut par conséquent être appliqué sans difficulté à l'échelle industrielle.

La réaction qui caractérise le procédé de l'invention est illustrée par le schéma B.

Le cétène, utilisé comme produit de départ, peut être obtenu à partir d'un produit commercial aisément disponible, le citral, suivant la méthode décrite par J. D. Wuest et al. [J. Org. Chem. 42, 2111 (1977)]. Le dérivé halomagnésien peut être préparé selon les méthodes courantes à partir de l'halogénure d'alkényle désiré et de magnésium, de préférence en milieu éthéré. A cet effet, on utilise de préférence le tétrahydrofuranne.

Le procédé de l'invention permet donc soit l'obtention directe de l'alpha-damascone (IIb), soit celle de son précurseur direct à double liaison terminale (IIa), ce dernier composé pouvant être transformé en alpha-damascone par traitement avec un agent isomérisant acide suivant l'une des méthodes connues [voir brevet suisse 537 452].

L'accès direct à l'alpha-damascone par la réaction de l'halogénure de 1-propényle sur le cétène I peut sembler à première vue plus économique que la voie qui consiste en la formation de l'intermédiaire IIa suivie d'isomérisation. En pratique, cependant, il s'est avéré que tel n'est pas le cas. En effet, les rendements observés lors de la réaction entre le cétène et l'halomagnésien montrent qu'il est plus avantageux de passer à travers l'intermédiaire IIa. D'autre part, au point de vue économique, il faut observer que les halogénures d'allyle sont disponibles sur le marché à un prix inférieur à celui des halogénures de 1-alkényle utiles, comme il est montré par une comparaison entre le prix respectif du chlorure d'allyle et du chlorure de 1-propényle par exemple. Enfin, la réaction entre les halogénures d'allyle et le cétène I procède de manière plus aisée que celle entre les halogénures de 1-propényle et ce même cétène, pour laquelle des conditions de réaction plus sévères doivent s'appliquer quant à la température et au temps de réaction. A cet effet, on peut mentionner que la réaction avec le chlorure d'allyle procède à température ambiante et l'addition est pratiquement complète en l'espace de quelques heures tandis que la réaction analogue avec le bromure de 1-propényle nécessite une température plus élevée et un temps de réaction prolongé.

Les cétones cycloaliphatiques IIa,b désirées sont généralement isolées du mélange réactionnel par hydrolyse du produit d'addition selon les techniques usuelles, par exemple au moyen d'une solution aqueuse concentrée de chlorure d'ammonium, suivie des traitements d'extraction, de neutralisation, lavage et distillation.

La transformation de la cétone IIa en l'alpha-damascone a lieu par isomérisation acide, par exemple au moyen d'acide p-toluènesulfonique. Dans ce cas aussi, le produit final désiré est obtenu par extraction à l'éther et traitement des extraits organiques.

Le procédé de l'invention est illustré de manière plus détaillée par les exemples suivants, dans lesquels les températures sont indiquées en degrés centigrades.

Exemple 1

Préparation de 1-[2,6,6-triméthyl-cyclohex-2-ényl]-but-3-ène-1-one

1,53 G (20 mM) de chlorure d'allyle dans 20 ml de tétrahydrofuranne anhydre ont été ajoutés à 20-30° à une suspension agitée de 0,48 g (20 mM) de magnésium en copeaux dans 5 ml de tétrahydrofuranne anhydre. Le mélange a été maintenu avec agitation pendant 1 h à température ambiante, puis 1,5 g (10 mM) de 2,6,6-triméthyl-cyclohex-2-énylcétène dans 10 ml de tétrahydrofuranne anhydre y ont été ajoutés à 20-30°. La réaction était légèrement exothermique et la couleur jaune de la solution du cétène disparaissait immédiatement. Le mélange de réaction a été maintenu sous agitation à température ambiante pendant 3 heures, concentré sous vide à 40-50° et hydrolysé avec un mélange d'eau et glace et de chlorure d'ammonium.
Après extraction à l'éther, les extraits éthérés ont été soumis aux traitements usuels de neutralisation avec une solution aqueuse d'HCl 1N, de bicarbonate de sodium et d'eau, puis ils ont été séchés sur $MgSO_4$ et concentrés.
Le produit brut (2 g) a été enfin distillé au moyen d'un appareil à boules (85-95°/1,33 Pa) pour fournir 1,61 g d'un mélange ayant un contenu en la cétone désirée de 80%.
Le mélange ainsi obtenu peut être employé directement pour la préparation d'alpha-damascone par traitement avec 30 mg d'acide p-toluènesulfonique.
Le 2,6,6-triméthyl-cyclohex-2-énylcétène, utilisé comme produit de départ dans le procédé décrit ci-dessus, a été préparé suivant la méthode décrite dans J. Org. Chem. 42, 2111 (1977) à partir du chlorure d'alpha-cyclogéranyle.

Exemple 2

Préparation d'alpha-damascone

2,42 G (20 mM) de bromure de 1-propényle dans 20 ml de tétrahydrofuranne anhydre ont été ajoutés à 40° à une suspension agitée de magnésium en copeaux (0,48 g ; 20 mM) dans 5 ml de tétrahydrofuranne. Le mélange réactionnel a été maintenu sous agitation à 40° pendant 1 h, puis 1,5 g de 2,6,6-triméthyl-cyclohex-2-énylcétène (10 mM) dans 10 ml de tétrahydrofuranne anhydre y ont été ajoutés goutte à goutte à température ambiante. Le mélange réactionnel a été maintenu sous agitation à 40° pendant 5 h, puis il a été concentré sous vide. Après hydrolyse avec un mélange d'eau-glace et une solution aqueuse concentrée de chlorure d'ammonium, on a extrait à l'éther et les extraits éthérés combinés ont été neutralisés avec de l'HCl 1N, du bicarbonate de sodium et de l'eau, puis séchés sur $MgSO_4$ et concentrés. Le produit brut obtenu (1,9 g) a été distillé dans un four à boules (95-105°/1,33 Pa) pour fournir 0,82 g d'alpha-damascone accompagnée par des traces de produits d'autre nature. Le rendement de la réaction exprimé en alpha-damascone était d'environ 29%.

**Revendications**

1. Procédé pour la préparation de cétones cycloaliphatiques de formule

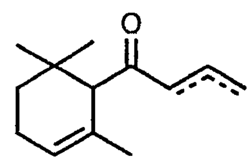

IIa,b

contenant une double liaison dans l'une des positions indiquées par les pointillés, caractérisé en ce qu'on additionne un halogénure d'alkényl-magnésium de formule

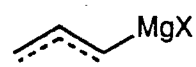

VI

contenant une double liaison dans l'une des positions indiquées par les pointillés et dans laquelle X représente un atome d'halogène, sur un cétène de formule

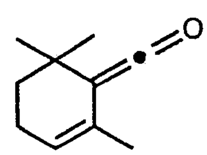

I

et hydrolyse le produit résultant.

2. Procédé selon la revendication 1, caractérisé en ce que l'addition s'effectue dans le tétrahydrofuranne.

3. Procédé selon la revendication 1, caractérisé en ce que l'halogénure d'alkényl-magnésium est le chlorure d'allyl-magnésium et que l'on obtient la 1-[2,6,6-triméthyl-cyclohex-2-ényl]-but-3-ène-1-one.

4. Procédé selon la revendication 3 caractérisé en ce qu'il comprend l'étape supplémentaire consistant en l'isomérisation acide du composé obtenu pour fournir l'alpha-damascone.

5. Procédé selon la revendication 1, caractérisé en ce que l'halogénure d'alkényl-magnésium est le bromure de 1-propényle-magnésium et que l'on obtient l'alpha-damascone.

**Claims**

1. Process for the preparation of cycloaliphatic ketones of formula

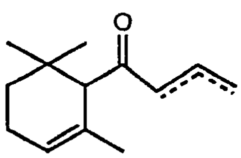

(IIa, b)

having a double bond in one of the positions indicated by the dotted lines, characterized in that an alkenyl-magnesium halide of formula

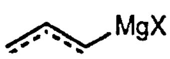

(VI)

having a double bond in one of the positions indicated by the dotted lines and where X represents a halogen atom, is added on a ketene of formula

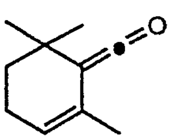

(I)

and the resulting product is hydrolyzed.

2. Process according to claim 1, characterized in that the addition is carried out in tetrahydrofuran.

3. Process according to claim 1, characterized in that the alkenyl-magnesium halide is allyl-magnesium chloride and that there is obtained 1-[2,6,6-trimethyl-cyclohex-2-enyl]-but-3-en-1-one.

4. Process according to claim 3, characterized by the additional step which consists in the acidic isomerization of the obtained compound to give α-damascone.

5. Process according to claim 1, characterized in that the alkenyl-magnesium halide is 1-propenyl-magnesium bromide and that there is obtained α-damascone.

**Patentansprüche**

1. Verfahren zur Herstellung von cycloaliphatischen Ketonen der Formel

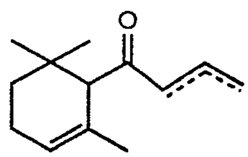

(IIa, b)

mit einer Doppel-bindung in einer der durch die gestrichelten Linien gezeichneten Stellungen, dadurch gekennzeichnet, daß man ein Alkenyl-magnesium Halogenid der Formel

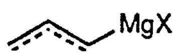

(VI)

mit einer Doppel-bindung in einer der durch die gestrichelten Linien gezeichneten Stellungen und worin X ein Halogen darstellt, zu einem Keten der Formel

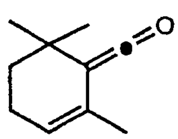

(I)

addiert und das so erhaltene Produkt hydrolisiert.

2. Verfahren gemäß Anspruch 1, dadurch gekennzeichnet, daß die Addition in Tetrahydrofuran durchgeführt wird.

3. Verfahren gemäß Anspruch 1, dadurch gekennzeichnet, daß man als Alkenyl-magnesium Halogenid Allyl-magnesium Chlorid verwendet und 1-[2,6,6,-Trimethyl-cyclohex-2-enyl]-but-3-en-1-on erhält.

4. Verfahren gemäß Anspruch 3, dadurch gekennzeichnet, daß man eine zusätzliche Stufe, die aus einer Säure Isomerisierung besteht durchführt, um α-Damascon zu erhalten.

5. Verfahren gemäß Anspruch 1, dadurch gekennzeichnet, daß man als Alkenyl-magnesium Halogenid 1-Propenyl-magnesium Bromid verwendet und α-Damascon erhält.

## ANNEXE

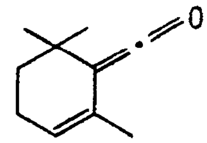

I

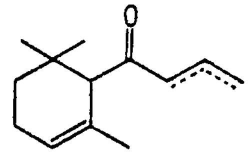

IIa,b

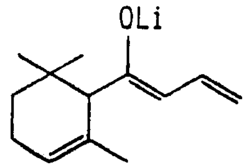

V

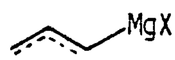

VI

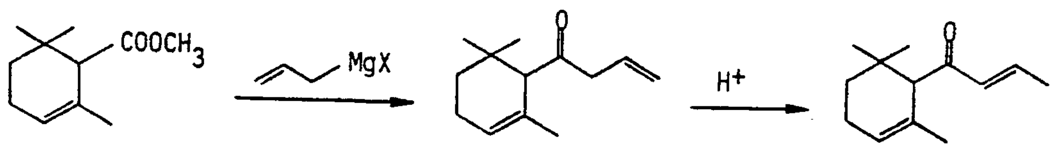


IV IIa IIb

X = halogène
HMPA = hexaméthyl triamidure de phosphore

[voir brevet suisse 563 951]

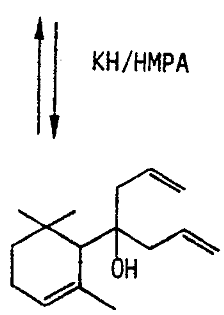


III

A

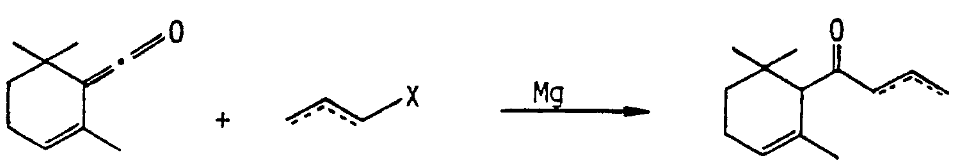


I IIa,b

B